(19) Europäisches Patentamt
European Patent Office
Office européen des brevets

(11) Publication number: **0 107 619**
**B1**

(12) **EUROPEAN PATENT SPECIFICATION**

(45) Date of publication of patent specification: **29.07.87**

(21) Application number: **83810442.0**

(22) Date of filing: **03.10.83**

(51) Int. Cl.⁴: **C 07 D 471/04,** A 61 K 31/435 // C07D231/38, C07D401/04, C07D407/04 ,(C07D471/04, 231:00, 221:00)

(54) **Dihydropyrazolo(3,4-b)pyridine derivatives and production thereof.**

(30) Priority: **05.10.82 JP 176763/82**

(43) Date of publication of application:
**02.05.84 Bulletin 84/18**

(45) Publication of the grant of the patent:
**29.07.87 Bulletin 87/31**

(84) Designated Contracting States:
**BE CH DE FR IT LI NL SE**

(56) References cited:
**US-A-3 857 849**

(73) Proprietor: **SHIONOGI & CO., LTD.**
**12, Dosho-machi 3-chome Higashi-ku**
**Osaka 541 (JP)**

(72) Inventor: **Adachi, Ikuo**
**3-34-5, Momoyama-dai**
**Suita-shi Osaka (JP)**
Inventor: **Yamamori, Teruo**
**1-8-39, Hikarigaoka**
**Takarazuka-shi Hyogo (JP)**
Inventor: **Ueda, Motohiko**
**7-16, Minamitakahama-cho**
**Suita-shi Osaka (JP)**
Inventor: **Sato, Hatsuo**
**657-11, Ikaruga-cho**
**Ikoma-gun Nara (JP)**

(74) Representative: **Tschudi, Lorenz et al**
**Bovard AG Patentanwälte VSP Optingenstrasse 16**
**CH-3000 Bern 25 (CH)**

EP 0 107 619 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to dihydropyrazolo[3,4-b]pyridine derivatives having calcium antagonistic activity comparable to those of the known 1,4-dihydropyridine derivatives such as nifedipine, nisoldipine, nicardipine, and so on. Moreover, the derivatives have slight negative inotropic effect as adverse effect which is a problem in the prior art.

The compounds having calcium-antagonistic action have been utilized in treatment of diseases in the circulatory system such as angina pectoris, hypertension, cerebrovascular dysfunction, arrhythmia, and so on, and their high therapeutic efficacy has been appreciated. Especially, a series of compounds named 1,4-dihydropyridine derivatives have been investigated as calcium-antagonists. As the known calcium-antagonists, for example, nifedipine (U.S. Patent No. 3,485,847), nisoldipine (Japanese Patent Publication No. 56—47185), 4-amino-1,4-dihydropyridine derivatives (Japanese Patent Publication No. 57—20306), 2-pyridyl-1,4-dihydropyridine derivatives (Japanese Unexamined Patent Publication No. 54—48796) are exemplified.

Meanwhile, the pyrazolodihydropyridine derivatives and their calcium-antagonistic action concerning the present invention have not yet been described in any literature.

The present invention relates to dihydropyrazolo[3,4-b]pyridine derivatives, production thereof, and pharmaceutical compositions thereof;

1) Dihydropyrazolo[3,4-b]pyridine derivatives represented by the general formula (I):

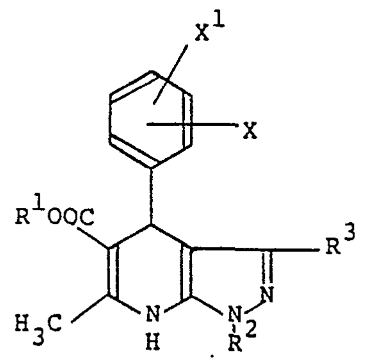

[wherein X and $X^1$ each is hydrogen, nitro, or halogen which may be located at the position or positions 2, 3, and/or 6;

$R^1$ is $C_1$—$C_4$ alkyl; $R^2$ is hydrogen, $C_1$—$C_4$ alkyl, $C_4$—$C_6$ cycloalkyl, or phenyl;

$R^3$ is hydrogen $C_1$—$C_8$ straight or branched chain alkyl, $C_3$—$C_7$ cycloalkyl which may be substituted by $C_1$—$C_3$ alkyl, unsubstituted phenyl, phenyl substituted by chlorine, trifluoromethyl, cyano, methoxy, methoxycarbonyl or ethoxycarbonyl, $C_7$—$C_9$ aralkyl, $C_1$—$C_4$ alkyloxycarbonyl, or 5- or 6- membered heterocyclic group containing an oxygen or nitrogen];

2) A process which comprises reacting a compound represented by the general formula (II):

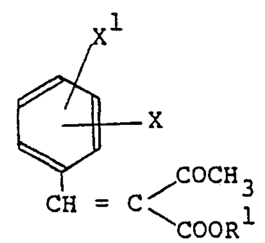

(II)

[wherein X, $X^1$ and $R^1$ each has the same significance as defined above] with a compound represented by the general formula (III):

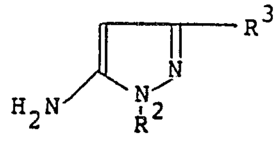

(III)

(wherein $R^2$ and $R^3$ each has the same significance as defined above] to yield the dihydropyrazolo[3,4-b]pyridine derivatives represented by the general formula (I).

3) Medicinal compositions comprising one or more members selected from the group consisting of dihydropyrazolo[3,4-b]pyridine derivatives represented by the general formula (I) and their pharmaceutical acceptable acid addition salts and pharmaceutical carriers.

The compounds (I) of the present invention are classified into calcium-antagonists having potent antihypertensive action and coronary vasodilating action and useful in treatment of diseases in the circulatory system such as angina pectoris, hypertension, cerebrovascular dysfunction, arrhythmia, and so on. The compounds (I) have slight negative inotropic effect with weak acute toxicities in mice.

The compounds (I) of the present invention are prepared by the Michael addition and concurrent cyclization reaction of heterocyclic groups with α,β-unsaturated ketones; the process for production thereof is also new in respect of providing new aromatic condensed dihydropyridines.

In the brief summary of the invention, definition relating to the general formulas (I)—(III);

the halogen means fluorine, chlorine, bromine, and iodine, particularly, chorine is preferred;

the $C_1$—$C_4$ alkyl means straight or branched chain lower alkyls; for example, methyl, ethyl, i-propyl and t-butyl;

the $C_4$—$C_6$ cycloalkyl includes cyclobutyl, cyclopentyl, and cyclohexyl;

The $C_1$—$C_8$ straight or branched chain alkyl includes methyl, ethyl, i-propyl, n-butyl, i-butyl, t-butyl, n-pentyl, 3-methylpent-3-yl, n-hexyl, n-heptyl, and n-octyl;

the $C_3$—$C_7$ cycloalkyl which may be substituted by $C_1$—$C_3$ alkyl includes cyclopropyl, cyclobutyl, cyclopentyl, cyclohexyl, cycloheptyl, 4-methylcyclohexyl, and 1-ethylcyclohexyl;

the phenyl group substituted by chlorine, trifluoromethyl, cyano, methoxy, methoxycarbonyl, or ethoxycarbonyl; includes for example, 3-chlorophenyl, 3,5-dichlorophenyl, 3-trifluoromethylphenyl, 3-cyanophenyl, 4-methoxyphenyl, 3-methoxycarbonylphenyl, or 3-ethoxycarbonylphenyl;

the $C_7$—$C_9$ aralkyl includes for example benzyl, phenethyl, phenylpropyl, and tolylmethyl;

the $C_1$—$C_4$ alkyloxycarbonyl includes for example methoxycarbonyl, ethoxycarbonyl, i-propoxycarbonyl, and t-butoxycarbonyl; the 5- or 6- membered heterocyclic group containing an oxygen or nitrogen includes for example α-pyridyl, β-furyl, and 1-methylimidazol-2-yl.

The objective compounds of the present invention (I) can be prepared easily by the reaction of α,β-unsaturated ketone reagents (II) with 5-aminopyrazole compounds (III) as shown in the following reaction sequence.

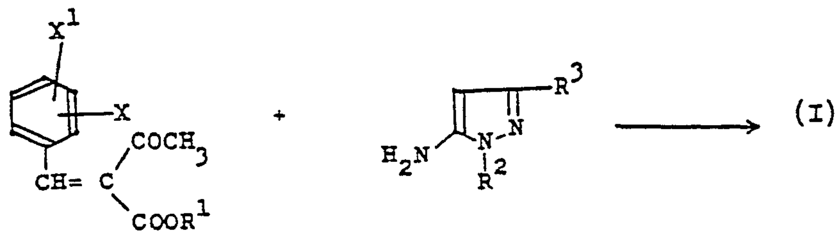

(II) (III)

[wherein X, $X^1$, $R^1$, $R^2$, and $R^3$ each has the same significance as defined above].

The above reaction is conducted in the absence or presence of solvents.

As the solvent used in the reaction, alcoholic solvents such as methanol, ethanol, i-propanol, t-butanol, and ethylene glycol; hydrocarbon solvents such as benzene, toluene, and xylene, ether solvents such as ether, tetrahydrofuran, dioxan, glyme, and diglyme; halogenohydrocarbons such as methylene chloride, chloroform, dichloroethane, and carbon tetrachloride; ester solvents such as ethyl acetate, and the like; and acetic acid, and dimethylformamide may be exemplified. If necessary, an acid or inorganic base may be used as catalyst. As the acid catalyst, inorganic acids such as sulfuric acid, hydrochloric acid, and phosphoric acid; organic acids such as p-toluenesulfonic acid, acetic acid, formic acid; and Lewis acid such as boron trifluoride, zinc chloride, aluminum chloride, magnesium chloride, and tin chloride, may be exemplified. As the organic base catalyst, triethylamine, pyridine, pyrrolidine, and piperidine may be exemplified. The reaction is conducted at room temperature or under heating (20—100°C), and usually terminates within a period of several hours to several days.

The starting 5-aminopyrazole compounds and α,β-unsaturated ketone reagents used in the reaction are prepared in the manner as shown below.

(1) Preparation of 5-aminopyrazole compounds (III)

The 5-aminopyrazole compounds (III) can be prepared according to three processes as shown in the following reaction sequence.

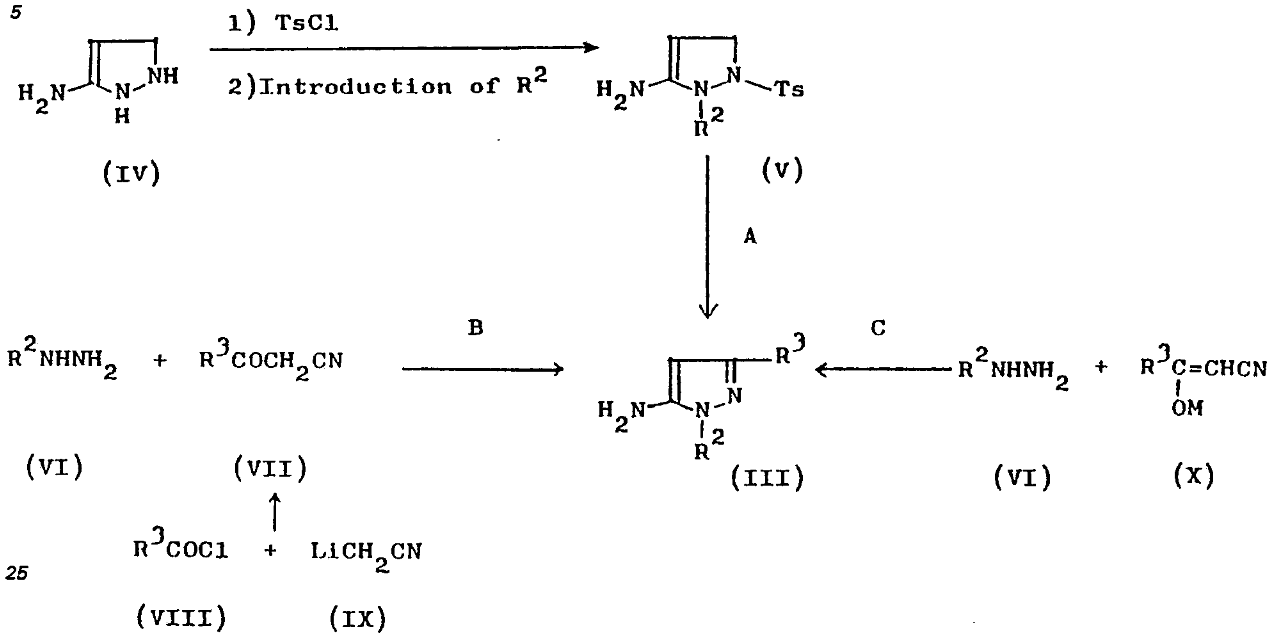


(IV) (V) (VI) (VII) (III) (VI) (X) (VIII) (IX)

[In the above sequence, $R^2$ and $R^3$ each has the same significance as defined above and M represents alkali metal.]

In the above reaction sequence the 5-aminopyrazole compounds (III) when $R^3$ is hydrogen may be prepared according to the process A from the compounds (IV) by tosylation and introduction of $R^2$ followed by elimination of the tosyl group with a base [Chem. Ber. *98* 3368 (1965)]. The compounds (III) when $R^3$ is neither hydrogen nor alkoxycarbonyl may be prepared according to the process B by cyclization reaction of hydrazine or methyl- or phenylhydrazines (VI) with a member of β-ketonitriles (VII). The β-ketonitriles (VII) are prepared by reaction of a member of acid chlorides (VIII) with the lithium acetonitrile (IX). The compounds (III) when $R^3$ is alkoxycarbonyl are prepared by cyclization reaction of a member of hydrazines (VI) with the alkali metal salt of alkyl 3-cyanopyruvates (X).

(2) Preparation of α,β-unsaturated ketone reagents (II)

The α,β-unsaturated ketone reagents (II) are prepared by the condensation of the aldehydes (XI) with the acetoacetic esters (XII) according to the following reaction sequence [J. Chem. Soc., *81* 1212 (1902), Chem. Ber. *29* 172 (1896), Ann. *278* 170 (1883), J. Chem. Soc., 3092 (1962)].

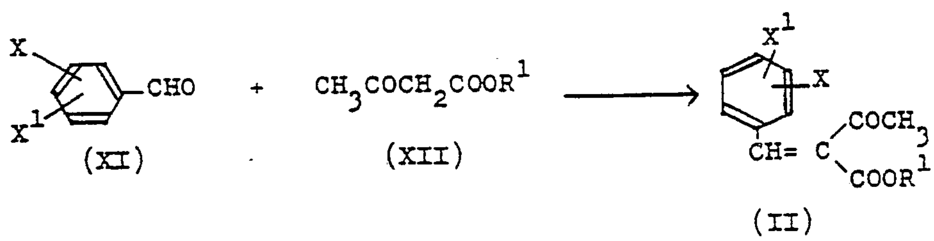

(XI) (XII) (II)

[wherein X, $X^1$, and $R^1$ each has the same significance as defined above].

The compounds of the present invention prepared from the 5-aminopyrazole compounds (III) by the reaction with the α,β-unsaturated ketone reagents (II) as mentioned above are exemplified as follows.

Ethyl 3 cyclopentyl-1,6-dimethyl-4-(3-nitrophenyl)-4,7-dihydropyrazolo[3,4-b]pyridine-5-carboxylate,
Ethyl 1,6-dimethyl-4-(3-nitrophenyl)-4,7-dihydropyrazolo[3,4-b]pyridine-5-carboxylate,
Methyl 1,6-dimethyl-4-(2-nitrophenyl)-4,7-dihydropyrazolo[3,4-b]pyridine-5-carboxylate,
Methyl 3-isopropyl-1,6-dimethyl-4-(3-nitrophenyl)-4,7-dihydropyrazolo[3,4-b]pyridine-5-carboxylate,
Methyl 3-(n-butyl)-1,6-dimethyl-4-(3-nitrophenyl)-4,7-dihydropyrazolo[3,4-b]pyridine-5-carboxylate,
Methyl 3-cyclobutyl-1,6-dimethyl-4-(3-nitrophenyl)-4,7-dihydropyrazolo[3,4-b]pyridine-5-carboxylate,
Methyl 3-cyclopentyl-1,6-dimethyl-4-(2-nitrophenyl)-4,7-dihydropyrazolo[3,4-b]pyridine-5-carboxylate,
Methyl 3-cyclopentyl-1,6-dimethyl-4-(3-nitrophenyl)-4,7-dihydropyrazolo[3,4-b]pyridine-5-carboxylate,
Isopropyl 3-cyclopentyl-1,6-dimethyl-4-(3-nitrophenyl)-4,7-dihydropyrazolo[3,4-b]pyridine-5-carboxylate,

Ethyl 3-cyclopentyl-1,6-dimethyl-4-(2-chlorophenyl)-4,7-dihydropyrazolo[3,4-b]pyridine-5-carboxylate,
Ethyl 3-cyclopentyl-1,6-dimethyl-4-(2,6-dichlorophenyl)-4,7-dihydropyrazolo[3,4-b]pyridine-5-carboxylate,
Methyl 3-cyclohexyl-1,6-dimethyl-4-(3-nitrophenyl)-4,7-dihydropyrazolo[3,4-b]pyridine-5-carboxylate,
Methyl 3 benzyl-1,6-dimethyl-4-(3-nitrophenyl)-4,7-dihydropyrazolo[3,4-b]pyridine-5-carboxylate,
Ethyl 3-phenyl-6-methyl-4-(3-nitrophenyl)-4,7-dihydropyrazolo[3,4-b]pyridine-5-carboxylate,
Ethyl 1,3-diphenyl-6-methyl-4-(3-nitrophenyl)-4,7-dihydropyrazolo[3,4-b]pyridine-5-carboxylate,
Methyl 3-phenyl-1,6-dimethyl-4-(3-nitrophenyl)-4,7-dihydropyrazolo[3,4-b]pyridine-5-carboxylate,
Ethyl 3-phenyl-1,6-dimethyl-4-(3-nitrophenyl)4,7-dihydropyrazolo[3,4-b]pyridine-5-carboxylate,
Methyl 3-phenyl-1,6-dimethyl-4-(2-nitrophenyl)-4,7-dihydropyrazolo[3,4-b]pyridine-5-carboxylate,
Ethyl 3-(3-chlorophenyl)-1,6-dimethyl-4-(3-nitrophenyl)-4,7-dihydropyrazolo[3,4-b]pyridine-5-carboxylate,
Methyl 3-(3,5-dichlorophenyl)-1,6-dimethyl-4-(3,nitrophenyl)-4,7-dihydropyrazolo[3,4-b]pyridine-5-carboxylate,
Ethyl 3-(3,5-dichlorophenyl)-1,6-dimethyl-4-(3-nitrophenyl)-4,7-dihydropyrazolo[3,4b]pyridine-5-carboxylate,
Ethyl 3-(3-trifluoromethylphenyl)-1,6-dimethyl-4-(3-nitrophenyl)-4,7-dihydropyrazolo[3,4-b]pyridine-5-carboxylate,
Ethyl 3-(3-cyanophenyl)-1,6-dimethyl-4-(3-nitrophenyl)-4,7-dihydropyrazolo[3,4-b]pyridine-5-carboxylate,
Ethyl 3-(3-methoxycarbonylphenyl)-1,6-dimethyl-4-(3-nitrophenyl)-4,7-dihydropyrazolo[3,4-b]pyridine-5-carboxylate,
Ethyl 3-(3-ethoxycarbonylphenyl)-1,6-dimethyl-4-(3-nitrophenyl)-4,7-dihydropyrazolo[3,4-b]pyridine-5-carboxylate,
Ethyl 3-(α-pyridyl)-1,6-dimethyl-4-(3-nitrophenyl)-4,7-dihydropyrazolo[3,4-b]pyridine-5-carboxylate,
Methyl 3-(β-furyl)-1,6-dimethyl-4-(3-nitrophenyl)-4,7-dihydropyrazolo[3,4-b]pyridine-5-carboxylate,
Methyl 3-methoxycarbonyl-1,6-dimethyl-4-(3-nitrophenyl)-4,7-dihydropyrazolo[3,4-b]pyridine-5-carboxylate,
Ethyl 3-ethoxycarbonyl-1,6-dimethyl-4-(3-nitrophenyl)-4,7-dihydropyrazolo[3,4-b]pyridine-5-carboxylate,
Methyl 3-isopropoxycarbonyl-1,6-dimethyl-4-(3-nitrophenyl)-4,7-dihydropyrazolo[3,4-b]pyridine-5-carboxylate,
Ethyl 3-(4-methoxyphenyl)-1,6-dimethyl-4-(3-nitrophenyl)-4,7-dihydropyrazolo[3,4-b]pyridine-5-carboxylate,
Methyl 1,3-dicyclopentyl-6-dimethyl-4-(3-nitrophenyl)-4,7-dihydropyrazolo[3,4-b]pyridine-5-carboxylate,
Methyl 3-isobutyl-1,6-dimethyl-4-(3-nitrophenyl)-4,7-dihydropyrazolo[3,4-b]pyridine-5-carboxylate,
Methyl 3-(t-butyl)-1,6-dimethyl-4-(3-nitrophenyl)-4,7-dihydropyrazolo[3,4-b]pyridine-5-carboxylate,
Methyl 3-(n-pentyl)-1,6-dimethyl-4-(3-nitrophenyl)-4,7-dihydropyrazolo[3,4-b]pyridine-5-carboxylate,
Methyl 3-(3-methylpent-3-yl)-1,6-dimethyl-4-(3-nitrophenyl)-4,7-dihydropyrazolo[3,4-b]pyridine-5-carboxylate,
Methyl 3-cyclopropyl-1,6-dimethyl-4-(3-nitrophenyl)-4,7-dihydropyrazolo[3,4-b]pyridine-5-carboxylate,
Methyl 3-cyclohexyl-1,6-dimethyl-4-(3-nitrophenyl)-4,7-dihydropyrazolo[3,4-b]pyridine-5-carboxylate,
Methyl 3-cycloheptyl-1,6-dimethyl-4-(3-nitrophenyl)-4,7-dihydropyrazolo[3,4-b]pyridine-5-carboxylate,
Methyl 3-(4-methylcyclohexyl)-1,6-dimethyl-4-(3-nitrophenyl)-4,7-dihydropyrazolo[3,4-b]pyridine-5-carboxylate,
Methyl 3-(1-ethylcyclohexyl)-1,6-dimethyl-4-(3-nitrophenyl)-4,7-dihydropyrazolo[3,4-b]pyridine-5-carboxylate,
Methyl 3-(1-methyl-imidazol-2-yl)-1,6-dimethyl-4-(3-nitrophenyl)-4,7-dihydropyrazolo[3,4-b]pyridine-5-carboxylate.

The compounds of the present invention have potent antihypertensive action and coronary vasodilating action which are based on calcium-antagonistic action; they also have platelet aggregation action, but they are advantageous in having no unpleasant systole inhibitory action undesired as pharmaceutical drug with lower toxicity. The biological tests of the compounds mentioned below were conducted as follows.

(The compounds)

(A): Nifedipine

(B): Methyl 3-cyclopentyl-1,6-dimethyl-4-(3-nitrophenyl)-4,7-dihydropyrazolo[3,4-b]pyridine-5-carboxylate

(C): Ethyl 3-cyclopentyl-1,6-dimethyl-4-(3-nitrophenyl)-4,7-dihydropyrazolo[3,4-b]pyridine-5-carboxylate hydrochloride

(1) Antihypertensive action

(Experimental method)

Female Spontaneously Hypertensive Rats (hereinafter abbreviated to as SHR) in which systolic pressure was about 160 mmHg were used without anesthetization. After SHR were warmed at 50°C for 2-3

minutes, systolic blood pressure was measured indirectly by the tail-cuff method using a Physiograph and Electrosphygmomanometer (DMP—4B and PE—300, Narco Biosystems, Inc., Houston). Each compound was intraperitoneally administered to SHR at a dose of 3 mg/kg body weight.

(Result)

TABLE 1

| Compounds | Maximum Hypotension (mmHg) | Duration of Effect (hours) |
|---|---|---|
| (A) | 45 | 6 |
| (B) | 66 | 16 |
| (C) | 42 | 14 |

(2) Coronary vasodilating action,
Negative inotropic action
(Experimental method)

The guinea-pigs (body weight: 400—800 g) of both sexes were hit on the head hard, and the arteria carotis was cut off and phlebotomized. The isolated heart was perfused at pressure of 50 cm $H_2O$ according to the Langendorff method [Basic Pharmacology & Therapeutics, 9 (4), 181 (1981)]. Krebs-Ringer bicarbonate solution containing 0.5% defibinated blood at 27°C was used as perfusate, into which a mixture of 95% oxygen adn 5% carbon dioxide was introduced continuously. The perfusion flow was led into a drop counter, and its increase or decrease was regarded as an indication of coronary vasodilation or vasoconstriction; the isometric contraction of apex was recorded along with the drop number of coronary perfusate on a Recticorder (RJG 3006, Nihon Koden) by way of an F—D pick-up (SB—1T, Nihon Koden). Each compound was administered into a rubber tube connected with an aorta cannula at a dose of 0.1 µg, 1 µg, and 10 µg.

(Result)

TABLE 2
Coronary vasodilating action

| Compounds | Perfusion Flow Change (%) | | |
|---|---|---|---|
| | 0.1µg | 1 µg | 10 µg |
| (A) | + 38 | + 100 | |
| (B) | + 26 | + 73 | + 180 |
| (C) | + 40 | + 79 | + 93 |

TABLE 3
Negative inotropic action

| Compounds | Change of Contractile Tension (%) | | |
|---|---|---|---|
| | 0.1 µg | 1 µg | 10µg |
| (A) | − 15 | − 57 | |
| (B) | 0 | 0 | 0 |
| (C) | 0 | 0 | 0 |

(3) Acute toxicity

(Experimental method)

In female DS mice (body weight: about 20 g), $LD_{50}$ value after the intravenous administration of the compounds was calculated by the Brownlee's up and down method [J. Am. Sat. As., *48* 262 (1953)].

(Result)

TABLE 4

| Compounds | $LD_{50}$ mg/Kg |
|---|---|
| (A) | 10.7 |
| (B) | 31.5 |
| (C) | 50.6 |

In consideration of the results mentioned above, the compounds of the present invention have remarkably potent antihypertensive and coronary vasodilating actions but slight negative inotropic action with lower acute toxicity, and so they can be utilized as drugs acting on the circulatory system with fewer adverse effects for men or animals.

The compounds of the present invention may be administered to men or animals orally or parenterally and formulated into various dosage forms according to the administration method. For example, tablets, capsules, pills, granules, fine granules, aqueous solution, emulsion, and so on may be prepared. In the pharmaceutical preparation, usual conventional carriers or diluents, such as lactose, sucrose, starch, cellulose, talc, magnesium stearate, magnesium oxide, calcium sulfate, powdered gum arabic, gelatin, sodium arginate, sodium benzoate, stearic acid, and the like may be used. As injection, a solution in water for injection, saline solution, Ringer solution, and so on, or a suspension in sesame oil may be used.

The compounds of the present invention may be administered at a dose of about 1—50 mg a day for an adult in oral administration and at a dose of about 0.5—20 mg in intravenous injection.

The present invention will be explained by the following Examples and Reference examples.

Example 1

Preparation of ethyl 3-cyclopentyl-1,6-dimethyl-4-(3-nitrophenyl)-4,7-dihydropyrazolo[3,4-b]pyridine-5-carboxylate

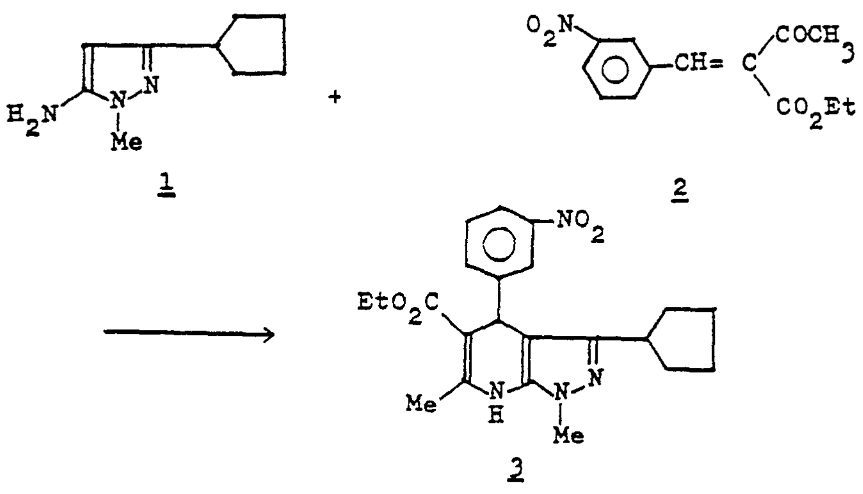

1 + 2 → 3

A mixture of 0.83 g (5 mmol) of 5-amino-3-cyclopentyl-1-methylpyrazole *1* and 1.32 g (5 mmol) of ethyl 3-nitrobenzylidene acetate *2* in 10 ml of t-butanol is heated at 80°C under nitrogen gas for 3 days. The mixture is concentrated under reduced pressure, and the resulting residue is dissolved in chloroform, washed with an aqueous sodium bicarbonate solution and then with an aqueous sodium chloride solution. The solution is dried with magnesium sulfate and chromatographed on a column of silica gel. The chloroform-ethyl acetate (20:1) fraction gives 2 g of the titled compound as an yellow oily material.

IR: $\nu_{max}^{Nujol}$ 3270, 3150, 1690, 1350 $cm^{-1}$

NMR: $\delta^{CDCl_3}$ 1.17 (3H, t), 1.00—2.80 (9H, m), 2.38, 3.67 (3H x 2, s), 4.03 (2H, q), 5.25 (1H, s), 7.13—8.10 (4H, m).

The titled compound (2 g) is converted into the hydrochloride on treatment with an ether-hydrochloric acid mixture, which is recrystallized from acetone to give 1.75 g of the hydrochloride. (Yield: 78.4%) m.p. 170—173°C

Elemental analysis
Calcd (%): C, 59.12; H, 6.09; N, 12.54 (for $C_{22}H_{26}N_4O_4.HCl$)
Found (%): C, 58.90; H, 6.10; N, 12.57
IR: $\nu_{max}^{Nujol}$ 2630, 2550, 1680, 1347 $cm^{-1}$
NMR: $\delta^{CDCl_3}$ 0.93—2.90 (9H, m), 1.17 (3H, t), 2.61 (3H, s) 4.03 (2H, q), 6.03 (1H), 5.22 (1H, s), 7.23—8.17 (4H, m)

Examples 2—42

In the same manner as in Example 1, the compounds described in Table 5 can be prepared. Tables 6 and 7 show the data of each product, i.e. physical constants, elemental analysis, IR spectra, and NMR spectra.

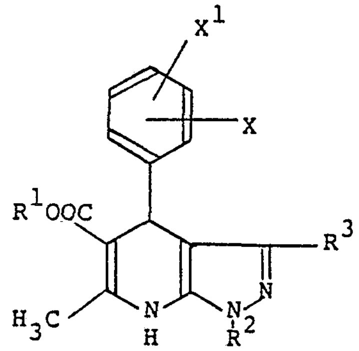

TABLE 5

| Ex. | $R^1$ | $R^2$ | $R^3$ | X . $X^1$ | Yield % |
|---|---|---|---|---|---|
| 2 | $C_2H_5$ | $CH_3$ | H | 3-$NO_2$,H | 47.4 |
| 3 | $CH_3$ | ″ | ″ | 2-$NO_2$,H | 42.1 |
| 4 | ″ | ″ | i-$C_3H_8$ | 3-$NO_2$,H | 92.6* |
| 5 | ″ | ″ | n-$C_4H_9$ | ″ | 96.7 |
| 6 | ″ | ″ | 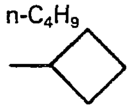 | ″ | 68.5 |
| 7 | ″ | ″ | 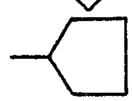 | 2-$NO_2$,H | 77.6 |
| 8 | ″ | ″ | ″ | 3-$NO_2$,H | 60.6 |
| 9 | i-$C_3H_8$ | ″ | ″ | ″ | 68.2* |
| 10 | $C_2H_5$ | ″ | ″ | 2-Cl,H | 59.0* |
| 11 | ″ | ″ | ″ | 2,6-$Cl_2$ | 15.7 |
| 12 | $CH_3$ | ″ | 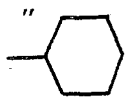 | 3-$NO_2$,H | 71.7* |
| 13 | ″ | ″ | 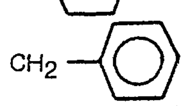 | ″ | 71.4* |
| 14 | $C_2H_5$ | H | 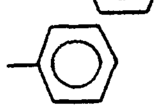 | 3-$NO_2$,H | 99.0 |
| 15 | ″ | 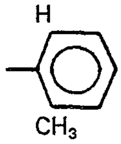 | ″ | ″ | 79.2 |
| 16 | $CH_3$ | $CH_3$ | 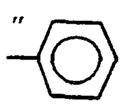 | 3-$NO_2$,H | 91.1 |

TABLE 5 continued

| Ex. | $R^1$ | $R^2$ | $R^3$ | X . $X^1$ | Yield % |
|---|---|---|---|---|---|
| 17 | $C_2H_5$ | " | " | " | 83.7 |
| 18 | $CH_3$ | " | " | 2-$NO_2$,H | 84.2 |
| 19 | $C_2H_5$ | " | 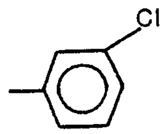 | 3-$NO_2$,H | 94.5 |
| 20 | $CH_3$ | " | 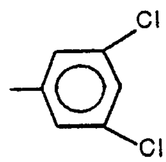 | " | 84.5 |
| 21 | $C_2H_5$ | " | " | " | 51.3 |
| 22 | " | " | 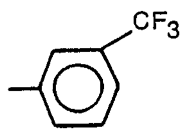 | " | 74.1 |
| 23 | " | " | 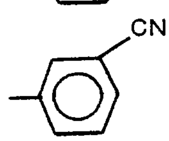 | " | 68.3 |
| 24 | " | " | 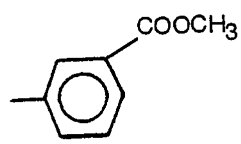 | " | 78.3 |
| 25 | " | " | 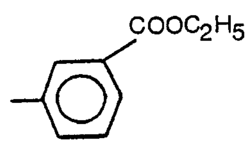 | " | 81.0 |
| 26 | $C_2H_5$ | $CH_3$ | 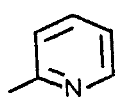 | 3-$NO_2$,H | 75.7 |
| 27 | $CH_3$ | " | 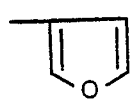 | " | 77.7 |
| 28 | " | " | $COOCH_3$ | " | 59.8 |
| 29 | $C_2H_5$ | " | $COOC_2H_5$ | " | 58.9 |
| 30 | $CH_3$ | " | COO-i-$C_3H_8$ | " | 56.5 |
| 31 | $C_2H_5$ | " | 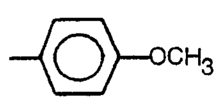 | " | 80.4 |
| 32 | $CH_3$ | 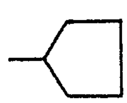 | 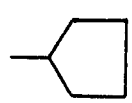 | " | 71.8 |

TABLE 5 continued

| Ex. | $R^1$ | $R^2$ | $R^3$ | X . $X^1$ | Yield % |
|---|---|---|---|---|---|
| 33 | $CH_3$ | $CH_3$ | $i\text{-}C_4H_9$ | $3\text{-}NO_2$,H | 59.0 |
| 34 | " | " | $t\text{-}C_4H_9$ | " | 38.8 |
| 35 | " | " | $n\text{-}C_5H_{11}$ | " | 85.7* |
| 36 | " | " | 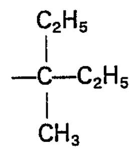 | " | 57.4* |
| 37 | " | " | 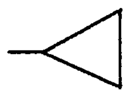 | " | 73.7 |
| 38 | " | " | 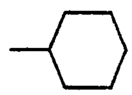 | $2\text{-}NO_2$,H | 79.6 |
| 39 | " | " | 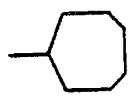 | $3\text{-}NO_2$,H | 95.2 |
| 40 | $CH_3$ | $CH_3$ | 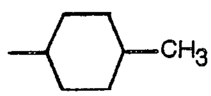 | $3\text{-}NO_2$,H | 53.2 |
| 41 | " | " | 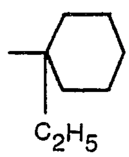 | " | 59.6* |
| 42 | " | " | 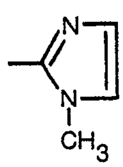 | " | 76.8 |

* Hydrochloride

TABLE 6

| Ex. | Appearance | Solvent in Recrystallization | M.P. (°C) | Molecular Formula | Elemental Analysis Calcd. C | H | N | Found C | H | N |
|---|---|---|---|---|---|---|---|---|---|---|
| 2 | YP | Ethyl acetate | 153—154 | $C_{17}H_{18}N_4O_4$ | 59.64 | 5.30 | 16.37 | 59.68 | 5.25 | 16.33 |
| 3 | " | Isopropanol | 213—214 | $C_{16}H_{16}N_4O_4$ | 58.53 | 4.91 | 17.07 | 58.68 | 4.89 | 17.14 |
| 4 | " | Methanol | 214—220 | $C_{20}H_{24}N_4O_4 \cdot HCl$ | 57.21 | 5.76 | 13.34 | 56.93 | 5.97 | 13.36 |
| 5 | OP | Ether | 129—132 | $C_{20}H_{24}N_4O_4$ | 62.48 | 6.29 | 14.58 | 62.52 | 6.31 | 14.46 |
| 6 | YP | Ethyl acetate | 183—185 | $C_{20}H_{22}N_4O_4$ | 62.81 | 5.80 | 14.65 | 62.63 | 5.83 | 14.58 |
| 7 | " | Ethanol | 208—213 | $C_{21}H_{24}N_4O_4$ | 63.62 | 6.10 | 14.13 | 63.50 | 6.15 | 14.10 |
| 8 | " | Isopropyl ether | 172—173 | $C_{21}H_{24}N_4O_4$ | 63.62 | 6.10 | 14.13 | 63.42 | 6.08 | 14.10 |
| 9 | CP | Isopropanol | 170—190 (dec.) | $C_{23}H_{25}N_4O_4 \cdot HCl \cdot \frac{1}{2}H_2O$ | 59.29 | 5.84 | 12.03 | 59.72 | 6.31 | 12.03 |
| 10 | " | Methanol-Acetone | 160—170 (dec.) | $C_{22}H_{26}N_3O_2Cl \cdot HCl$ | 60.55 | 6.24 | 9.63 | 60.63 | 6.31 | 9.75 |
| 11 | " | Isopropyl ether | 146—148 | $C_{22}H_{25}N_3O_2Cl_2 \cdot \frac{1}{2}H_2O$ | 59.60 | 5.91 | 9.47 | 59.56 | 6.04 | 9.41 |
| 12 | YP | Methanol | 195—230 (dec.) | $C_{22}H_{26}N_4O_4 \cdot HCl$ | 59.13 | 6.09 | 12.54 | 58.76 | 6.09 | 12.50 |
| 13 | CP | Methylene chloride-Ether | 124—126 | $C_{23}H_{22}N_4O_4 \cdot HCl \cdot \frac{1}{2}H_2O$ | 59.54 | 5.22 | 12.07 | 59.35 | 5.36 | 11.83 |
| 14 | YP | Benzene | 233—234 | $C_{22}H_{20}N_4O_4$ | 65.33 | 4.99 | 13.86 | 65.23 | 4.83 | 13.93 |
| 15 | " | Ethyl acetate | 214—215 | $C_{28}H_{24}N_4O_6$ | 69.99 | 5.03 | 11.66 | 70.28 | 5.09 | 11.68 |
| 16 | OP | " | 209—210 | $C_{22}H_{20}N_4O_4$ | 65.23 | 4.99 | 13.86 | 65.42 | 4.91 | 13.89 |
| 17 | YN | Methylene chloride-Ether | 157—158 | $C_{23}H_{22}N_4O_4$ | 66.01 | 5.30 | 13.39 | 65.94 | 5.16 | 13.33 |
| 18 | OP | Chloroform | 205—206 | $C_{22}H_{20}N_4O_4\frac{1}{2}H_2O$ | 63.91 | 5.12 | 13.55 | 64.17 | 5.06 | 13.64 |

TABLE 6 continued

| Ex. | Appearance | Solvent in Recrystallization | M.P. (°C) | Molecular Formula | Elemental Analysis Calcd. C | Calcd. H | Calcd. N | Found C | Found H | Found N |
|---|---|---|---|---|---|---|---|---|---|---|
| 19 | YP | Ethanol | 214—216 | $C_{23}H_{21}N_4O_4Cl$ | 61.00 | 4.67 | 12.37 | 61.03 | 4.43 | 12.38 |
| 20 | " | Tetrahydrofuran-Ethanol | 263—264 | $C_{22}H_{18}N_4O_4Cl_2$ | 55.82 | 3.83 | 11.84 | 55.80 | 3.82 | 11.74 |
| 21 | " | Methylene chloride-Ether | 222—224 | $C_{23}H_{20}N_4O_4Cl_2$ | 56.68 | 4.14 | 11.50 | 56.32 | 4.22 | 11.43 |
| 22 | " | Methanol | 217—218 | $C_{24}H_{21}N_4O_4F_3$ | 59.25 | 4.35 | 11.52 | 59.31 | 4.39 | 11.56 |
| 23 | YN | " | 211—214 | $C_{24}H_{21}N_5O_4$ | 65.00 | 4.77 | 15.79 | 64.87 | 4.89 | 15.67 |
| 24 | OP | Ethanol | 182—183 | $C_{25}H_{24}N_4O_6$ | 63.01 | 5.08 | 11.76 | 62.93 | 5.11 | 11.80 |
| 25 | YN | Methanol | 225—227 | $C_{26}H_{26}N_4O_6$ | 63.66 | 5.34 | 11.42 | 63.42 | 5.36 | 11.39 |
| 26 | YP | Isopropanol | 213—214 | $C_{22}H_{21}N_5O_4$ | 63.00 | 5.05 | 16.70 | 63.16 | 4.94 | 16.63 |
| 27 | " | Ethanol | 195—198 | $C_{20}H_{18}N_4O_5$ | 60.91 | 4.60 | 14.21 | 60.85 | 4.70 | 14.08 |
| 28 | " | Ethyl acetate | 206—209 | $C_{18}H_{18}N_4O_6$ | 55.95 | 4.70 | 14.50 | 55.91 | 4.71 | 14.40 |
| 29 | YN | " | 133—135 | $C_{20}H_{22}N_4O_6$ | 57.96 | 5.35 | 13.52 | 57.29 | 5.34 | 12.97 |
| 30 | YP | " | 180—182 | $C_{20}H_{22}N_4O_6$ | 57.96 | 5.35 | 13.52 | 57.73 | 5.36 | 13.30 |
| 31 | " | " | 159—161 | $C_{24}H_{24}N_4O_5$ | 64.27 | 5.39 | 12.49 | 64.33 | 5.42 | 12.48 |
| 32 | OP | Ethanol | 175—177 | $C_{25}H_{30}N_4O_4$ | 66.66 | 6.71 | 12.44 | 66.68 | 6.68 | 12.44 |
| 33 | YP | Ethyl acetate | 110—115 | $C_{20}H_{24}N_4O_4$ | 62.48 | 6.29 | 14.58 | 62.12 | 6.12 | 14.76 |
| 34 | " | " | 153—154 | $C_{20}H_{24}N_4O_4 \cdot \frac{1}{2}H_2O$ | 61.06 | 6.40 | 14.24 | 61.25 | 6.52 | 13.88 |
| 35 | " | Ethanol | 140—155 | $C_{21}H_{26}N_4O_4 \cdot HCl$ | 58.00 | 6.26 | 12.88 | 58.03 | 6.37 | 12.71 |

TABLE 6 continued

| Ex. | Appear-ance | Solvent in Recrystallization | M.P. (°C) | Molecular Formula | Elemental Analysis | | | | | |
|---|---|---|---|---|---|---|---|---|---|---|
| | | | | | Calcd. | | | Found | | |
| | | | | | C | H | N | C | H | N |
| 36 | CP | Acetone | 172—175 | $C_{22}H_{28}N_4O_4 \cdot HCl$ | 58.86 | 6.51 | 12.48 | 58.59 | 6.47 | 12.46 |
| 37 | YP | Ethyl ether | 100—102 | $C_{19}H_{20}N_4O_4$ | 61.94 | 5.47 | 15.21 | 61.84 | 5.56 | 15.05 |
| 38 | " | Methyl cyanide | 215—219 | $C_{22}H_{26}N_4O_4$ | 64.37 | 6.39 | 13.65 | 64.21 | 6.31 | 13.52 |
| 39 | " | Ethyl ether | 199—200 | $C_{23}H_{28}N_4O_4$ | 65.07 | 6.65 | 13.20 | 65.13 | 6.77 | 13.10 |
| 40 | " | Isopropanol | 197—198 | $C_{23}H_{28}N_4O_4$ | 65.07 | 6.65 | 13.20 | 65.09 | 6.60 | 13.05 |
| 41 | PL | Ethanol | 175—176 | $C_{24}H_{30}N_4O_4 \cdot HCl$ | 60.69 | 6.58 | 11.80 | 60.35 | 6.43 | 11.77 |
| 42 | YN | Methanol | 207—208 (dec.) | $C_{20}H_{20}N_6O_4 \cdot CH_3OH$ | 57.00 | 5.92 | 19.00 | 57.10 | 5.48 | 19.05 |

* YP = Yellow prisms, CP = Colorless prisms, OP = Orange prisms, YN = Yellow needles, PL = Colorless plates

TABLE 7

| Ex. | IR ($v_{max}^{cm^{-1}}$) NH | | CO | NO$_2$ | NMR ($\delta^{CDCl_3}$) |
|---|---|---|---|---|---|
| 2 | 3340 | | 1690 | 1343 | 1.10 (3H, t), 2.45 (3H, s), 3.70 (3H, s) 4.00 (2H, q), 5.30 (1H, s), 7.00 (1H, s), 7.20 (1H, bs), 7.30—8.10 (4H, m) |
| 3 | 3280 | 3175 | 1680 | 1350 | 2.45 (3H, s), 3.40 (3H, s), 3.70 (3H, s), 5.70 (1H, s), 6.80 (1H, bs), 7.30—7.85 (4H, m) |
| 4 | 3430 | 3290 | 1690 | 1350 | 0.90 (3H, d), 1.10 (3H, d), 1.20 (3H, t), 2.40 (3H, s), 2.20—2.80 (1H, m), 3.70 (3H, s), 4.05 (2H, m), 5.30 (1H, s), 6.90 (1H, bs), 7.35—8.10 (4H, m) |
| 5 | 3350 | | 1693 | 1345 | 0.53—1.53 (7H, m), 2.20 (2H, m) 2.40 (3H, s), 3.59 (3H, s), 3.68 (3H, s) 5.25 (1H, s), 7.44 (1H, bs), 7.23—8.13 (4H, m) |
| 6 | 3375 | | 1705 | 1350 | 2.10—3.07 (7H, m), 2.37 (3H, s), 3.56 (3H, s) 3.65 (3H, s), 5.16 (1H, s), 7.37—7.90 (5H, m) |
| 7 | 3290 | | 1673 | 1353 | 0.87—3.10 (9H, m), 2.32 (3H, s), 3.32 (3H, s) 3.67 (3H, s), 5.62 (1H, s), 7.10—7.83 (4H, m) |
| 8 | 3375 | | 1700 | 1380 | 1.17—2.83 (9H, m), 2.27 (3H, s), 3.58 (3H, s), 3.67 (3H, s), 5.25 (1H, s), 6.70 (1H, bs), 7.27—8.10 (4H, m) |
| 9 | 2560 | | 1693 | 1353 | *1.03 (3H, d), 1.25 (3H, d), 1.53—2.60 (9H, m), 2.38 (3H, s), 3.65 (3H, s), 4.90 (2H, q), 5.23 (1H, s), 7.40—7.93 (5H, m) |
| 10 | 2360 | | 1701 | | *1.17 (3H, t), 1.60—2.80 (9H, m), 2.38 (3H, s), 3.52 (3H, s), 4.02 (2H, q), 5.62 (1H, s), 7.13—8.10 (5H, m) |
| 11 | 3470 | 3280 | 1670 | | 1.07 (3H, t), 0.80—3.27 (9H, m), 2.30 (3H, s), 3.57 (3H, s), 3.99 (2H, q), 6.08 (1H, s), 6.90—7.80 (5H, m) |
| 12 | 2495 | | 1699 | 1352 | *0.77—2.57 (11H, m), 2.42 (3H, s), 3.62 (3H, s), 3.70 (3H, s), 5.30 (1H, s), |
| 13 | 3200 | | 1690 | 1350 | 2.30 (3H, s), 3.52 (3H, s), 3.60 (5H, s), 5.00 (1H, s) 6.73—8.23 (4H, m) |
| 14 | 3200 | 3110 | 1705 | 1347 | **1.15 (3H, t), 2.50 (3H, s), 4.03 (2H, q), 6.17 (1H, bs), 6.45 (1H, s), 7.10—8.17 (9H, m), 10.33 (1H, bs) |

TABLE 7 continued

| Ex. | IR ($\nu_{max}^{cm^{-1}}$) NH | CO | NO$_2$ | NMR ($\delta^{CDCl_3}$) |
|---|---|---|---|---|
| 15 | 3360 | 1698 | 1345 | **1.13 (3H, t), 2.43 (3H, t) 4.02 (2H, q), 5.62 (1H, s), 6.90 (1H, bs), 7.13—8.03 (9H, m) |
| 16 | 3350 | 1665 | 1373 | **2.42 (3H, s), 3.50 (3H, s), 3.80 (3H, s), 5.52 (1H, s), 7.03—7.93 (14H, m), 9.57 (1H, bs) |
| 17 | 3280 | 1678 | 1350 | 1.18 (3H, t), 2.43 (3H, s), 3.77 (3H, s), 4.07 (2H, q), 5.50 (1H, s), 6.78 (1H, bs), 7.13—8.03 (9H, m) |
| 18 | 3315 | 1675 | 1375 | 2.32 (3H, s), 3.35 (3H, s), 3.80 (3H, s), 6.05 (1H, s), 7.07—7.77 (9H, m), 9.60 (1H, bs) |
| 19 | 3340 | 1690 | 1346 | 1.20 (3H, t), 2.45 (3H, s), 3.80 (3H, s), 4.10 (2H, q), 5.50 (1H, s), 6.55 (1H, bs), 7.10—8.10 (9H, m) |
| 20 | 3355 | 1683 | 1350 | 2.40 (3H, s), 3.53 (3H, s), 3.83 (3H, s), 5.47 (1H, s), 7.20—8.00 (8H, m), 9.60 (1H, bs) |
| 21 | 3350 | 1692 | 1350 | 1.25 (3H, s), 2.43 (3H, s), 3.80 (3H, s), 4.12 (2H, q), 5.48 (1H, s), 7.03—8.10 (7H, m) |
| 22 | 3340 | 1692 | 1348 | 1.22 (3H, t), 2.45 (3H, s), 3.80 (3H, s), 4.08 (2H, q), 5.50 (1H, s), 6.45 (1H, bs), 7.17—8.02 (7H, m) |
| 23 | 3350 | 1692 | 1348 | 1.20 (3H, t), 2.45 (3H, s), 3.82 (3H, s), 4.07 (2H, q), 5.50 (1H, s), 7.12 (1H, bs), 7.18—8.05 (8H, m) |
| 24 | 3330 | 1723 1694 | 1343 | 1.22 (3H, t), 2.43 (3H, s), 3.80 (3H, s), 3.93 (3H, s), 4.09 (2H, q), 5.60 (1H, s), 7.18 (1H, bs), 7.12—8.22 (8H, m) |
| 25 | 3270 | 1712 1668 | 1350 | 1.20 (3H, t), 1.40 (3H, t), 2.47 (3H, s), 3.83 (3H, s), 4.09 (2H, q), 4.40 (2H, q), 5.60 (1H, s), 6.60 (1H, bs), 7.07—8.23 (8H, m) |
| 26 | 3360 | 1695 | 1355 | 1.20 (3H, t), 2.45 (3H, s), 3.70 (3H, s), 4.05 (2H, q), 5.80 (1H, s) 6.80 (1H, bs), 7.00—8.70 (8H, m) |
| 27 | 3360 | 1701 | 1350 | 2.39 (3H, s), 3.64 (3H, s), 3.74 (3H, s), 5.36 (1H, s), 6.50—8.63 (8H, m) |

TABLE 7 continued

| Ex. | IR ($v_{max}^{cm^{-1}}$) NH | | CO | | $NO_2$ | NMR ($\delta^{CDCl_3}$) |
|---|---|---|---|---|---|---|
| 28 | 3320 | | 1725 | 1700 | 1355 | 2.39 (3H, s), 3.58 (3H, s), 3.75 (3H, s), 3.78 (3H, s), 5.50 (1H, s), 7.26—7.88 (7H, m), 7.60 (1H, bs) |
| 29 | 3220 | 3120 | 1735 | 1693 | 1350 | 1.20 (3H, t), 1.30 (3H, t), 2.42 (3H, s), 3.80 (3H, s), 4.07 (2H, q), 4.29 (2H, q), 5.58 (1H, s), 7.45 (1H, bs), 7.18—8.15 (4H, m) |
| 30 | 3370 | | 1725 | 1693 | 1350 | 1.20 (3H, d), 1.32 (3H, d), 2.38 (3H, s), 3.63 (3H, s), 3.78 (3H, s), 5.17 (1H, m), 5.58 (1H, s), 7.35—8.02 (4H, m), 8.07 (1H, bs) |
| 31 | 3290 | | 1630 | | 1350 | 1.18 (3H, t), 2.43 (3H, s), 3.77 (6H, s), 4.07 (2H, q) 5.50 (1H, s), 6.67—8.07 (9H, m) |
| 32 | 3350 | | 1695 | | 1340 | 1.22—2.20 (16H, m), 2.40 (3H, s), 2.62 (1H, m), 3.58 (3H, s), 4.35 (1H, m), 5.25 (1H, s), 4.35 (1H, bs), 7.30—8.13 (4H, m) |
| 33 | 3220 | | 1690 | | 1347 | 0.70, 0.84 (6H, d), 1.82 (3H, m), 2.40, 3.57, 3.67 (9H, s), 5.20 (1H, s), 7.08 (1H, bs), 7.62 (4H, m) |
| 34 | 3300 | | 1700 | | 1350 | 1.08 (9H, s), 2.42, 3.72, 3.78 (9H, s), 5.45 (1H, s), 6.57 (1H, bs), 7.67 (4H, m) |
| 35 | 2560 | | 1701 | | 1347 | 0.53—1.62(9H,m),2.22(2H,m),2.37,3.55,3.63(9H,s),5.20(1H,s),7.12—8.20(4H,m) 2.73 (1H, bs) |
| 36 | 2640 | | 1710 | | 1350 | 0.33, 0.58 (6H, d), 1.07, 2.34, 3.68, 3.73 (12H, s), 1.49 (4H, m), 5.32 (1H, s) 7.13 (1H, bs), 7.65 (4H, m) |
| 37 | 3225 | | 1695 | | 1350 | 0.27—1.67(5H,m),2.40,3.57,3.60(9H,s),5.28(1H,s),7.02(1H,bs),7.15—8.07(4M,m) |
| 38 | 3320 | | 1685 | | 1360 | 0.70—2.87(11H,m),2.37,3.50,3.67(9H,s),5.87(1H,s),7.12(1H,bs),7.00—7.83(4H,m) |
| 39 | 3355 | | 1700 | | 1350 | 1.07—2.62 (13H, m), 2.40, 3.58, 3.67 (9H, s), 5.25 (1H, s), 6.97 (1H, bs), 7.28—8.12 (4H, m) |
| 40 | 3340 | | 1695 | | 1340 | 0.70—2.35 (10H, m), 0.84 (3H, d), 2.41, 3.59, 3.68 (9H, s), 5.27 (1H, s), 6.79 (1H, bs) 7.30—8.15 (4M, m) |
| 41 | 2600 | | 1666 | | 1344 | 0.33 (3H, t), 0.73—2.53 (12H, m), 2.36, 3.68, 3.73 (9H, s), 5.30 (1H, s), 6.88 (1H, bs) 7.11—8.01 (4H, m) |
| 42 | | | 1667 | | 1347 | 2.65, 3.18, 3.44, 3.60 (12H, s), 5.38 (1H, s), 6.82—7.88 (6H, m), 8.65 (1H, bs) |

* Free base **in DMSO-d6

Example 43

| Component (Tablet) | |
|---|---|
| Ethyl 3-cyclopentyl-1,6-dimethyl-4-(3-nitrophenyl)-4,7-dihydropyrazolo[3,4-b]pyridine-5-carboxylate | 10 mg |
| Corn starch | 50 mg |
| Gelatin | 7.5 mg |
| Avicel (microcrystalline cellulose) | 25 mg |
| Magnesium stearate | 2.5 mg |
| | Total 950 mg |

The above composition is formulated into one tablet.

Reference example 1

i) Preparation of 5-amino-3-isopropyl-1-methylpyrazole

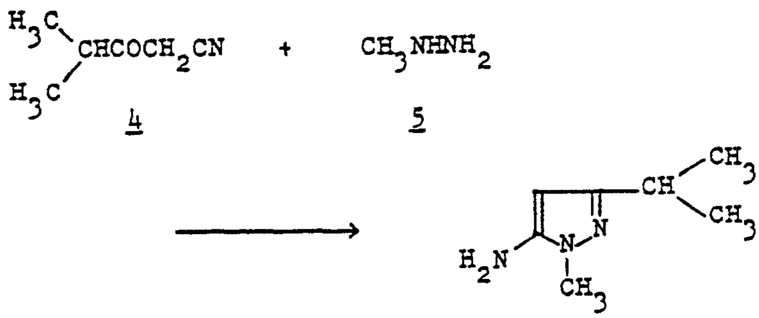

4 5

6

A mixture of 8.0 g (72 mmol) of 1-cyano-3-methyl-2-butanone *4* and 3.4 g (73.8 mmol) of methylhydrazine *5* in 2 ml of ethanol is stirred at room temperature for an hour, and concentrated under reduced pressure. The resulting residue is chromatographed on silica gel. The chloroform fraction is recrystallized from carbon tetrachloride to give 8.48 g (84.6% yield) of the titled compound as colorless prisms.

m.p. 111—112°C

NMR $\delta^{CDCl_3}$ 1.20 (6H, d), 2.60—3.10 (1H, m), 3.40 (2H, bs), 3.60 (3H, s), 5.30 (1H, s).

ii) Preparation of 1-cyano-3-methyl-2-butanone

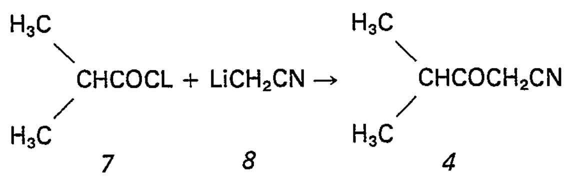

7 8 4

To a solution (100 ml) of 0.2 mol of n-butyllithium in anhydrous tetrahydrofuran is dropwise added a solution of 8.2 g (0.2 mmol) of acetonitrile in 12 ml of tetrahydrofuran under nitrogen gas at −70°C within 30 minutes; after 2 hours, a solution of 10.65 g (0.1 mmol) of isobutyrylchloride in 18 ml of tetrahydrofuran is dropwise added thereto. After an hour, the reaction mixture is acidified with 10% hydrochloric acid and extracted with ether, and the extract is washed with an aqueous sodium chloride solution, dried with magnesium sulfate, and evaporated. The residue is distilled to give 8.05 g (72.5% yield) of the titled compound.

m.p. 62—65°C

NMR: $\delta^{CDCl_3}$ 1.2 (6H, d), 2.6—3.1 (1H, m), 3.5 (2H, m).

Reference Examples 2—19

In the same manner as in Reference example 1, the compounds described in Table 8 can be prepared.

TABLE 8

| Ref. Ex. | $R^2$ | $R^3$ | Yield (%) | M.P. (°C) | NMR ($\delta^{CDCl}{}_{3}$3) |
|---|---|---|---|---|---|
| 2 | $CH_3$ | n-$C_4H_9$ | 89.1 | | 0.70—2.00 (7H, m), 2.60 (2H, t), 3.54 (3H, s), 5.29 (1H, s) |
| 3 | $CH_3$ | 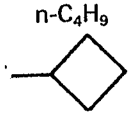 | 68.2 | 117—118 | 1.50—3.33 (7H, m), 3.54 (3H, s), 3.60 (2H, bs), 5.38 (1H, s) |
| 4 | $CH_3$ | 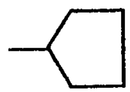 | 74.4 | 149—150 | 1.33—2.20 (8H, m), 2.60—3.30 (1H, m), 3.57 (5H, s), 5.32 (1H, s) |
| 5 | $CH_3$ | 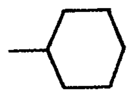 | 7.71 | 173—174 | 0.90—2.77 (11H, m), 3.43 (2H, bs), 3.57 (3H, s), 5.32 (1H, s) |
| 6 | $CH_3$ | 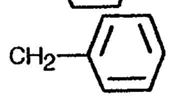 | 89.2 | 130—131 | 3.40 (2H, bs), 3.53 (3H, s), 3.78 (2H, s), 5.22 (1H, s), 7.23 (5H, s) |
| 7 | H | 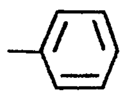 | 94.3 | 110—111 | 4.75 (2H, bs), 5.80 (1H, s), 7.10—7.77 (5H, m) |
| 8 | 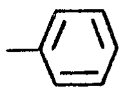 | ,, | 78.0 | 130—131 | 5.83 (1H, s), 7.20—7.80 (5H, m) |
| 9 | $CH_3$ | ,, | 92.4 | 130—131 | 3.58 (3H, s), 3.50 (2H, bs), 5.73 (1H, s), 7.15—7.78 (5H, m) |
| 10 | $CH_3$ | 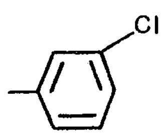 | 31.0 | 127—128 | 3.65 (3H, s), 5.80 (1H, s), 7.10—7.80 (4H, m) |
| 11 | $CH_3$ | 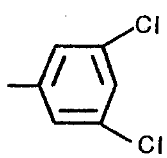 | 99.1 | 155—156 | 3.63 (3H, s), 3.63 (2H, bs), 5.72 (1H, s), 7.07—7.67 (3H, m) |
| 12 | $CH_3$ | 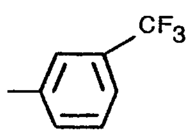 | 62.2 | 91—92 | 3.58 (2H, bs), 3.68 (3H, s), 5.85 (1H, s), 7.30—8.07 (4H, m) |

TABLE 8 CONTINUED

| Ref. Ex. | $R^2$ | $R^3$ | Yield (%) | M.P. (°C) | NMR ($\delta^{CDCl_3}$) |
|---|---|---|---|---|---|
| 13 | $CH_3$ | 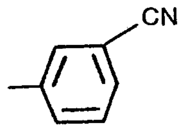 | 79.1 | 179—181 | 3.55 (2H, bs), 3.69 (3H, s), 5.81 (1H, s), 7.48—7.85 (4H, m) |
| 14 | $CH_3$ | 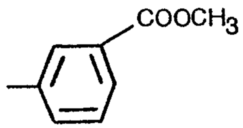 | 76.8 | 101—102 | 3.63 (2H, bs), 3.68 (3H, s), 3.90 (3H, s), 5.88 (1H, s), 7.42—8.37 (4H, m) |
| 15 | $CH_3$ | bl 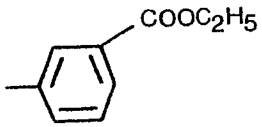 | 77.9 | 113—114 | 1.38 (3H, t), 3.62 (2H, bs), 3.68 (3H, s), 4.38 (2H, q), 5.88 (1H), 7.40—8.36 (4H, m) |
| 16 | $CH_3$ | 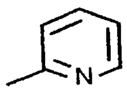 | 53.5 | 186—187 | 3.65 (2H, bs), 3.70 (3H, s), 6.15 (11H, s), 7.00—8.00 (4H, m) |
| 17 | $CH_3$ | 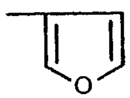 | 14.2 | 118—120 | 3.52 (2H, bs), 3.65 (3H, s), 5.63 (1H, s), 6.68—7.69 (3H, m) |
| 18 | $CH_3$ | 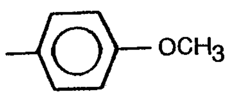 | 75.1 | 140—141 | 3.65 (3H, s), 3.78 (3H, s), 5.75 (1H, s), 6.73—7.67 (4H, m) |
| 19 | 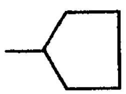 | 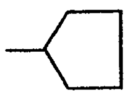 | 71.5 | 92—93 | 1.2—2.33 (16H, m), 2.97 (1H, m), 3.42 (2H, bs), 4.35 (1H, m), 5.33 (1H, s) |

Reference Example 20

Preparation of ethyl 5-amino-1methyl-pyrazole-3-carboxylate

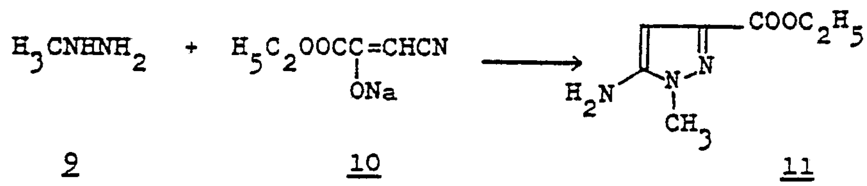

9 10 11

A mixture of 10 g (61.3 mmol) of the sodium salt of ethyl 3-cyanopyruvate *10* and 9.0 g (61.3 mmol) of methylhydrazine sulfate *9* in 100 ml of methanol is stirred at room temperature for 3 days, and then concentrated under reduced pressure. To the resulting residue are added an aqueous sodium bicarbonate solution and an aqueous sodium chloride solution, and the mixture is extracted 6 times with chloroform, dried with magnesium sulfate, and chromatographed on a column of silica gel. The ethyl acetate eluate gives 6.54 g (62.9% yield) of the titled compound *11* as an yellow oil.

NMR: $\delta^{CDCl_3}$ 1.35 (3H, t), 3.71 (3H, s), 3.76 (2H, bs), 4.35 (2H, q), 6.03 (1H, s).

Reference Example 21

Preparation of methyl 5-amino-1-methylpyrazole-3-carboxylate

To a solution of 48 mg (2.1 mmol) of sodium in 40 ml of methanol is added 2.0 mg (11.8 mmol) of 5-amino-3-ethoxy-carbonyl-1-methylpyrazole, and the mixture is refluxed over night, and distilled under reduced pressure. To the resulting residue are added a small amount of water and sodium chloride, and the mixture is extracted 6 times with chloroform. The extract is dried with magnesium sulfate, chromatographed on a column of silica gel and eluted with ethyl acetate to give 1.37 g (74.1% yield) of the titled compound.

m.p. 101—102°C

NMR: $\delta^{CDCl_3}$ 3.71 (3H, s), 3.86 (3H, s), 3.90 (2H, bs), 6.05 (1H, s).

Reference Example 22

Preparation of isopropyl 5-amino-1-methylpyrazole-3-carboxylate

In the same manner as in Reference example 19, the titled compound can be prepared. (Yield: 72.9%). m.p. 86—87°C

NMR: $\delta^{CDCl_3}$ 1.37 (6H, d), 3.72 (3H, s), 3.75 (2H, bs), 5.23 (1H, m)

Reference Example 23

Preparation of methyl 2-nitrobenzylidene acetate

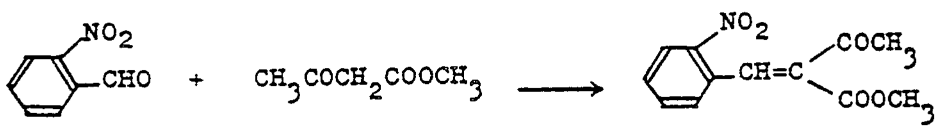

12 13 14

To 40 ml of benzene are added 11.6 g (0.1 mol) of methyl acetoacetate *13*, 15 g (0.1 mol) of 2-nitrobenzaldehyde *12*, 3 ml of acetic acid, and 0.8 ml of piperidine, and the mixture is kept at room temperature for 3 days, after which is added 12 g (0.1 mol) of magnesium sulfate thereto. The reaction mixture is stirred for 4 days, and filtered. Benzene is distilled off, and the residue is recrystallized from ethanol to give 22.5 g (90.0% yield) of the titled compound *14* as colorless prisms.

m.p. 100—101°C.

NMR: $\delta^{CDCl_3}$ 2.47 (3H, s), 3.60 (3H, s), 7.23—8.37 (4H, m).

**Claims**

1. Dihydropyrazolo[3,4-b]pyridine derivatives represented by the general formula:

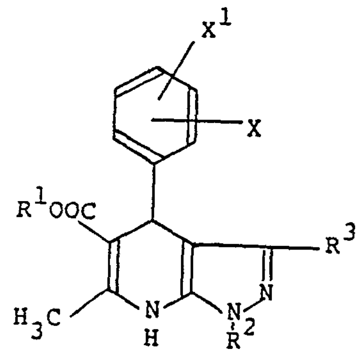

(I)

wherein X and $X^1$ each is hydrogen, nitro, or halogen which may be located at the position or positions 2, 3, and/or 6;

$R^1$ is $C_1$—$C_4$ alkyl;

$R^2$ is hydrogen, $C_1$—$C_4$ alkyl, $C_4$—$C_6$ cycloalkyl, or phenyl;

$R^3$ is hydrogen, $C_1$—$C_8$ straight or branched chain alkyl,

$C_3$—$C_7$ cycloalkyl which may be substituted by $C_1$—$C_3$ alkyl, phenyl substituted by chlorine, trifluormethyl, cyano, methoxy, methoxycarbonyl or ethoxycarbonyl, $C_7$—$C_9$ aralkyl, $C_1$—$C_4$ alkoxycarbonyl, or 5- or 6-membered heterocyclic group containing an oxygen or nitrogen and their acid addition salts.

2. A compound claimed in claim 1, wherein X is nitro;
$X^1$ is hydrogen; $R^1$ is $C_1$—$C_4$ alkyl; $R^2$ is $C_1$—$C_4$ alkyl;
$R^3$ is $C_1$—$C_8$ straight or branched chain alkyl, or $C_3$—$C_7$ cycloalkyl.

3. A compound claimed in claim 1, namely, methyl 3-(n-butyl)-1,6-dimethyl-4-(3-nitrophenyl)-4,7-dihydropyrazolo[3,4-b]pyridine-5-carboxylate.

4. A compound claimed in claim 1, namely, methyl 3-(i-butyl)-1,6-dimethyl-4-(3-nitrophenyl)-4,7-dihydropyrazolo[3,4-b]pyridine-5-carboxylate.

5. A compound claimed in claim 1, namely, methyl 3-cyclobutyl-1,6-dimethyl-4-(3-nitrophenyl)-4,7-dihydropyrazolo[3,4-b]pyridine-5-carboxylate.

6. A compound claimed in claim 1, namely, methyl 3-cyclopentyl-1,6-dimethyl-4-(2-nitrophenyl)-4,7-dihydropyrazolo[3,4-b]pyridine-5-carboxylate.

7. A compound claimed in claim 1, namely, methyl 3-cyclopentyl-1,6-dimethyl-4-(3-nitrophenyl)-4,7-dihydropyrazolo[3,4-b]pyridine-5-carboxylate.

8. A compound claimed in claim 1, namely, ethyl 3-cyclo-1,6-dimethyl-4-(3-nitrophenyl)-4,7-dihydropyrazolo[3,4-b]pyridine-5-carboxylate.

9. A compound claimed in claim 1, namely, isopropyl 3-cyclopentyl-1,6-dimethyl-4-(3-nitrophenyl-4,7)-dihydropyrazolo[3,4-b]pyridine-5-carboxylate.

10. A compound claimed in claim 1, namely, methyl 3-cyclohexyl-1,6-dimethyl-4-(3-nitrophenyl)-4,7-dihydropyrazolo[3,4-b]pyridine-5-carboxylate.

11. A compound claimed in claim 1, namely, methyl 3-cyclohexyl-1,6-dimethyl-4-(3-nitrophenyl)-4,7-dihydropyrazolo[3,4-b]pyridine-5-carboxylate.

12. A compound claimed in claim 1, namely, methyl 3-cycloheptyl-1,6-dimethyl-4-(3-nitrophenyl)-4,7-dihydropyrazolo[3,4-b]pyridine-5-carboxylate.

13. A process for producing dihydropyrazolo(3,4-b)pyridine derivatives of the formula (I) according to claim 1 which comprises reacting a compound represented by the general formula:

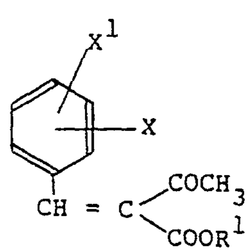

(II)

wherein X, $X^1$, and $R^1$ each has the same significance as defined in claim 1, with a compound represented by the general formula:

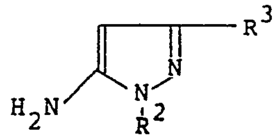

(III)

wherein $R^2$ and $R^3$ each has the same significance as defined in claim 1.

14. Medicinal compositions comprising one or more members selected from the group consisting of dihydropyrazolo[3,4-b]pyridine derivatives of the formula (I) according to claim 1 and their pharmaceutical acceptable acid addition salts and pharmaceutical carriers.

**Patentansprüche**

1. Dihydropyrazolo[3,4-b]pyridin-Derivate der allgemeinen Formel

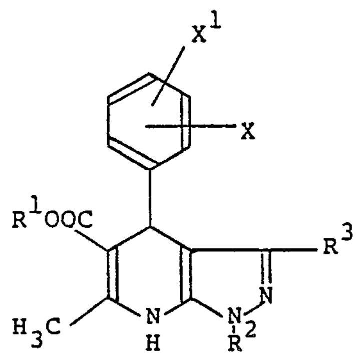

(I)

worin

X und $X^1$ jeder Wasserstoff, Nitro oder Halogen ist, welche sich in der Stellung oder den Stellungen 2, 3 und/oder 6 befinden können;

$R^1$ $C_1$—$C_4$-Alkyl ist;

$R^2$ Wasserstoff, $C_1$—$C_4$-Alkyl, $C_4$—$C_6$-Cycloalkyl oder Phenyl ist;

$R^3$ Wasserstoff, $C_1$—$C_8$ gerad- oder verzweigtkettiges Alkyl, $C_3$—$C_7$-Cycloalkyl, welches durch $C_1$—$C_3$-Alkyl substituiert sein kann, Phenyl, substituiert durch Chlor, Trifluormethyl, Cyano, Methoxy, Methoxycarbonyl oder Ethoxycarbonyl, $C_7$—$C_9$-Aralkyl, $C_1$—$C_4$-Alkoxycarbonyl oder eine 5- oder 6-gliedrige heterocyclische Gruppe, die Sauerstoff oder Stickstoff enthält, ist und ihre Säureadditionssalze.

2. Verbindung gemäss Anspruch 1, worin X Nitro ist; $X^1$ Wasserstoff ist; $R^1$ $C_1$—$C_4$-Alkyl ist; $R^2$ $C_1$—$C_4$-Alkyl ist; $R^3$ $C_1$—$C_8$ gerad- oder verzweigtkettiges Alkyl oder $C_3$—$C_7$-Cycloalkyl ist.

3. Verbindung gemäss Anspruch 1, nämlich Methyl-3-(n-butyl)-1,6-dimethyl-4-(3-nitrophenyl)-4,7-dihydropyrazolo-[3,4-b]-pyridin-5-carboxylat.

4. Verbindung gemäss Anspruch 1, nämlich Methyl-3-(i-butyl)-1,6-dimethyl-4-(3-nitrophenyl)-4,7-dihydropyrazolo-[3,4-b]-pyridin-5-carboxylat.

5. Verbindung gemäss Anspruch 1, nämlich Methyl-3-cyclo-butyl-1,6-dimethyl-4-(3-nitrophenyl)-4,7-dihydropyrazolo-[3,4-b]-pyridin-5-carboxylat.

6. Verbindung gemäss Anspruch 1, nämlich Methyl-3-cyclopentyl-1,6-dimethyl-4-(2-nitrophenyl)-4,7-dihydropyrazolo-[3,4-b]-pyridin-5-carboxylat.

7. Verbindung gemäss Anspruch 1, nämlich Methyl-3-cyclopentyl-1,6-dimethyl-4-(3-nitrophenyl)-4,7-dihydropyrazolo-[3,4-b]-pyridin-5-carboxylat.

8. Verbindung gemäss Anspruch 1, nämlich Ethyl-3-cyclopentyl-1,6-dimethyl-4-(3-nitrophenyl)-4,7-dihydropyrazolo-[3,4-b]-pyridin-5-carboxylat.

9. Verbindung gemäss Anspruch 1, nämlich Isopropyl-3-cyclopentyl-1,6-dimethyl-4-(3-nitrophenyl)-4,7-dihydropyrazolo-[3,4-b]-pyridin-5-carboxylat.

10. Verbindung gemäss Anspruch 1, nämlich Methyl-3-cyclohexyl-1,6-dimethyl-4-(3-nitrophenyl)-4,7-dihydropyrazolo-[3,4-b]-pyridin-5-carboxylat.

11. Verbindung gemäss Anspruch 1, nämlich Methyl-3-cyclohexyl-1,6-dimethyl-4-(3-nitrophenyl)-4,7-dihydropyrazolo-[3,4-b]-pyridin-5-carboxylat.

12. Verbindung gemäss Anspruch 1, nämlich Methyl-3-cycloheptyl-1,6-dimethyl-4-(3-nitrophenyl)-4,7-dihydropyrazolo-[3,4-b]-pyridin-5-carboxylat.

13. Verfahren zur Herstellung von Dihydropyrazolo-[3,4-b]-pyridin-Derivaten der Formel I gemäss Anspruch 1, dadurch gekennzeichnet, dass eine Verbindung der allgemeinen Formel

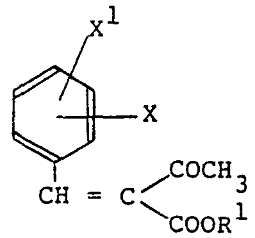

(II)

worin X, $X^1$ und $R^1$ je die gleiche Bedeutung wie im Patentanspruch 1 haben, mit einer Verbindung der allgemeinen Formel

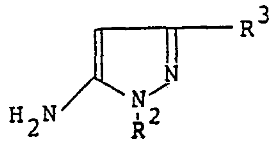

(III)

worin $R^2$ und $R^3$ je die gleiche Bedeutung, wie im Patentanspruch 1 definiert, haben, umgesetzt wird.

14. Medizinische Zusammensetzungen, dadurch gekennzeichnet, dass sie eines oder mehrere Mitglieder der Gruppe, bestehend aus den Dihydropyrazolo[3,4-b]pyridin-Derivaten der Formel I gemäss Anspruch 1 und ihren pharmazeutisch annehmbaren Säureadditionssalzen und pharmazeutische Träger enthalten.

**Revendications**

1. Dérivés de la dihydropyrazolo[3,4-b]pyridine représentés par la formule générale:

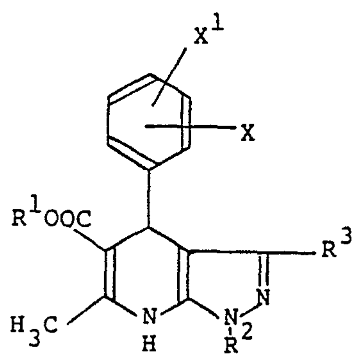

(I)

dans laquelle

X et $X^1$ sont chacun l'hydrogène, un groupe nitro ou l'halogène pouvant se situer à la position ou aux positions 2, 3 et/ou 6;

$R^1$ est un groupe alkyle en $C_1$—$C_4$;

$R^2$ est l'hydrogène, des groupes alkyle en $C_1$—$C_4$, cycloalkyle en $C_4$—$C_6$ ou phényle;

$R^3$ est l'hydrogène, un groupe alkyle à chaîne ramifiée ou linéaire en $C_1$—$C_8$, un groupe cycloalkyle en $C_3$—$C_7$ qui peut être substitué par un groupe alkyle en $C_1$—$C_3$, un groupe phényle substitué par le chlore, des groupes trifluorméthyle, cyano, méthoxy, méthoxycarbonyle ou éthoxycarbonyle, aralkyle en $C_7$—$C_9$, alkoxycarbonyle en $C_1$—$C_4$ ou un groupe hétérocyclique à 5 ou 6 membres contenant un atome d'oxygène ou d'azote et leurs sels d'acides.

2. Composé selon la revendication 1, dans lequel X est un groupe nitro; $X^1$ est l'hydrogène; $R^1$ est un groupe alkyle en $C_1$—$C_4$; $R^2$ est un groupe alkyle en $C_1$—$C_4$; $R^3$ est un groupe alkyle à chaîne ramifiée ou linéaire en $C_1$—$C_8$ ou un groupe cycloalkyle en $C_3$—$C_7$.

3. Composé selon la revendication 1, c'est-à-dire le 3-(n-butyl)-1,6-diméthyl-4-(3-nitrophényl)-4,7-dihydropyrazolo[3,4-b]pyridine-5-carboxylate de méthyle.

4. Composé selon la revendication 1, c'est-à-dire le 3-(i-butyl)-1,6-diméthyl-4-(3-nitrophényl)-4,7-dihydropyrazolo[3,4-b]pyridine-5-carboxylate de méthyle.

5. Composé selon la revendication 1, c'est-à-dire le 3-cyclobutyl-1,6-diméthyl-4-(3-nitrophényl)-4,7-dihydropyrazolo[3,4-b]pyridine-5-carboxylate de méthyle.

6. Composé selon la revendication 1, c'est-à-dire le 3-cyclopentyl-1,6-diméthyl-4-(3-nitrophényl)-4,7-dihydropyrazolo[3,4-b]pyridine-5-carboxylate de méthyle.

7. Composé selon la revendication 1, c'est-à-dire le 3-cyclopentyl-1,6-diméthyl-4-(3-nitrophényl)-4,7-dihydropyrazolo[3,4-b]pyridine-5-carboxylate de méthyle.

8. Composé selon la revendication 1, c'est-à-dire le 3-cyclopentyl-1,6-diméthyl-4-(3-nitrophényl-)4,7-dihydropyrazolo[3,4-b]pyridine-5-carboxylate de d'éthyle.

9. Composé selon la revendication 1, c'est-à-dire le 3-cyclopentyl-1,6-diméthyl-4-(3-nitrophényl)-4,7-dihydropyrazolo[3,4-b]pyridine-5-carboxylate d'isopropyle.

10. Composé selon la revendication 1, c'est-à-dire le 3-cyclohexyl-1,6-diméthyl-4-(3-nitrophényl)-4,7-dihydropyrazolo[3,4-b]pyridine-5-carboxylate de méthyle.

11. Composé selon la revendication, c'est-à-dire le 3-cyclohexyl-1,6-diméthyl-4-(3-nitrophényl)-4,7-dihydropyrazolo[3,4-b]pyridine-5-carboxylate de méthyle.

12. Composé selon la revendication 1, c'est-à-dire le 3-cycloheptyl-1,6-diméthyl-4-(3-nitrophényl)-4,7-dihydropyrazolo[3,4-b]pyridine-5-carboxylate de méthyle.

13. Procédé de préparation des dérivés de la dihydropyrazolo[3,4-b]pyridine de la formule (I) selon la revendication 1 consistant à faire réagir un composé représenté par la formule générale

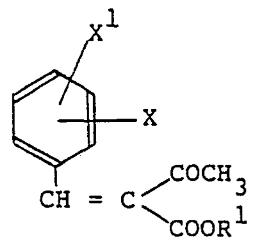

(II)

dans laquelle X, $X^1$ et $R^1$ ont chacun la même signification que celle définie dans la revendication 1, avec un composé représenté par la formule générale:

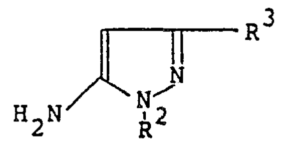

(III)

dans laquelle $R^2$ et $R^2$ ont chacun la même signification que celle définie dans la revendication 1.

14. Compositions pharmaceutiques comprenant un ou plusieurs membres choisis dans le groupe constituant les dérivés de la dihydropyrazolo[3,4-b]pyridine de la formule (I) selon la revendication 1 et leurs sels d'acide pharmaceutiquement acceptables et agents pharmaceutiques.